(19) Europäisches Patentamt
European Patent Office
Office européen des brevets

(11) **EP 4 517 317 A1**

(12) **EUROPEAN PATENT APPLICATION**
published in accordance with Art. 153(4) EPC

(43) Date of publication:
05.03.2025 Bulletin 2025/10

(21) Application number: 23796077.8

(22) Date of filing: 10.04.2023

(51) International Patent Classification (IPC):
*G01N 27/333* (2006.01)     *G01N 27/416* (2006.01)
*G01N 37/00* (2006.01)

(52) Cooperative Patent Classification (CPC):
G01N 27/333; G01N 27/416; G01N 37/00

(86) International application number:
PCT/JP2023/014603

(87) International publication number:
WO 2023/210332 (02.11.2023 Gazette 2023/44)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 28.04.2022 JP 2022074918

(71) Applicant: CANON KABUSHIKI KAISHA
Tokyo 146-8501 (JP)

(72) Inventors:
• MIURA, Jun
  Tokyo 146-8501 (JP)
• YAMAMOTO, Takeshi
  Tokyo 146-8501 (JP)
• MIYAZAKI, Keiji
  Tokyo 146-8501 (JP)

• FUKATSU, Makoto
  Tokyo 146-8501 (JP)
• TANAKA, Masanori
  Tokyo 146-8501 (JP)
• MAEDA, Harunobu
  Tokyo 146-8501 (JP)
• ENOKIDO, Fuka
  Tokyo 146-8501 (JP)
• MATSUKAWA, Akihisa
  Tokyo 146-8501 (JP)
• IKAMI, Yasukazu
  Tokyo 146-8501 (JP)
• ABE, Katsuichi
  Tokyo 146-8501 (JP)

(74) Representative: TBK
Bavariaring 4-6
80336 München (DE)

(54) **MICROCHANNEL DEVICE**

(57)     A thin microchannel device capable of performing stable measurement is provided. In a microchannel device using a porous substrate, the microchannel device has a channel region using a porous body, the channel region partially has a region A where a component having ion selectivity is present in a pore in the porous substrate, and, in the region A, the component having ion selectivity is present in the pore at one plane X side of the porous substrate and an other plane Y side of the porous substrate is a channel in which an empty pore is present, a working electrode is provided on a surface at the plane X side of the region A so as to at least partially overlap a component having ion selectivity, and a reference electrode is provided in a region B other than the region A of the channel region, and the region A and the region B are formed in the same porous substrate.

FIG. 2B

## Description

Technical Field

The present invention relates to a microchannel device in which electrodes are formed by using a channel of a porous substrate.

Background Art

In recent years, development of a microchannel device that allows an analysis in biochemistry to be efficiently (in minute amount, quickly, simply) performed in a single chip by using a microchannel with a microsize has become a focus of attention in a wide range of fields. A wide range of fields includes not only the research of biochemistry but also medicine, innovative drug development, health care, environment, food, and the like. Among them, a paper-based paper microanalytical chip has many advantages in light weight, low cost, no need of power supply, and high disposability as compared to an existing device. For this reason, it is expected as an inspection device for medical care in developing countries, backwoods, or disaster sites where medical equipment is not enough, airports where the spread of infectious diseases needs to be checked near water, or the like. Since a paper-based paper microanalytical chip is low in cost and easy to handle, the paper-based paper microanalytical chip has become a focus of attention also as a health care device with which his or her own health condition can be managed or monitored.

As an example of the paper microanalytical chip, in PTL 1, two paper substrates each having an electrode printed on its surface are used, an ion selective membrane is held therebetween, and a predetermined solution is dispensed to each paper substrate, with the result that a configuration similar to an ion selective electrode and a reference electrode each having internal liquid is formed. With this configuration, it is possible to perform an ion electrode method, and it is possible to measure the ion concentration of a sample. NPL 1 describes a method of forming an ion selective electrode and a reference electrode on a single paper substrate, and it is possible to measure the ion concentration of a sample with the ion electrode method.

Citation List

Patent Literature

PTL 1: U.S. Patent Application Publication No. 2016/033438

Non Patent Literature

NPL 1: Nipapan Ruecha, Orawon Chailapakul, Koji Suzuki and Daniel Chitterio "Fully Inkjet-Printed Paper-Based Potentiometric Ion-Sensing Devices" Analytical chemistry August 29, 2017 Published, 89, pp. 10608-10616

Summary of Invention

Technical Problem

However, with the method described in PTL 1, a porous substrate layer is present between a working electrode and an ion selective membrane, and there is a possibility that a measurement value is unstable when air bubbles are formed at an electrode interface at the time of filling liquid substitute for an internal solution. Furthermore, since the ion selective membrane is sandwiched by two paper substrates, not only the thickness of the electrode and the ion selective membrane but also a thickness equivalent to two sheets of porous substrate is needed as the thickness of an analytical chip.

With the method described in NPL 1, a sufficient amount of sample cannot permeate into a region of paper substrate in which the ion selective membrane is formed, so a contact area between a sample and the ion selective membrane is not sufficiently provided and becomes unstable, with the result that measurement accuracy can be influenced.

An object of one aspect of the present disclosure is to obtain stable measurement with a configuration in which a working electrode and an ion selective membrane are caused to contact with each other, miniaturization resulting from formation of all the electrodes on a single porous substrate, and stable measurement by sufficiently ensuring a contact area between the ion selective membrane and the sample without increasing an electrode area.

Solution to Problem

An aspect of the present invention provides a microchannel device using a porous substrate. The microchannel device has a channel region using a porous body, the channel region partially has a region A where a component having ion selectivity is present in a pore in the porous substrate, and, in the region A, the component having ion selectivity is present in the pore at one plane X side of the porous substrate and an other plane Y side of the porous substrate is a channel in which an empty pore is present, a working electrode is provided on a surface at the plane X side of the region A, a reference electrode is provided in a region B other than the region A of the channel region, and the region A and the region B are formed in the same porous substrate.

Advantageous Effects of Invention

According to the aspect of the present disclosure, it is possible to provide a small microchannel device with high measurement stability.

Brief Description of Drawings

[Fig. 1] Fig. 1 is a top view of a microchannel device M1 after a channel wall 11 is formed in Example 1.
[Fig. 2A] Fig. 2A is a schematic view of the microchannel device M1 after various electrodes are formed in Example 1.
[Fig. 2B] Fig. 2B is a schematic view of the microchannel device M1 after various electrodes are formed in Example 1.
[Fig. 3A] Fig. 3A is a schematic view that illustrates permeation of a sample in the microchannel device M1 of Example 1.
[Fig. 3B] Fig. 3B is a schematic view that illustrates permeation of a sample in the microchannel device M1 of Example 1.
[Fig. 4A] Fig. 4A is a schematic view of a microchannel device M2 after channel walls 12 are formed in Example 2.
[Fig. 4B] Fig. 4B is a schematic view of the microchannel device M2 after the channel walls 12 are formed in Example 2.
[Fig. 5A] Fig. 5A is a schematic view of the microchannel device M2 after various electrodes are formed in Example 2.
[Fig. 5B] Fig. 5B is a schematic view of the microchannel device M2 after various electrodes are formed in Example 2.
[Fig. 6A] Fig. 6A is a schematic view of an existing configuration example in which a working electrode is covered with an ion selective membrane.
[Fig. 6B] Fig. 6B is a schematic view of the existing configuration example in which the working electrode is covered with the ion selective membrane.

Description of Embodiments

Hereinafter, an example embodiment of the present invention will be described with reference to the drawings. The following embodiment is illustrative and is not intended to limit the present invention to the details of the embodiment. In the following drawings, components not needed to describe the embodiment are omitted from the drawings.
A microchannel device according to the present invention is a microchannel device using a porous substrate and has a channel region surrounded by a channel wall.
Examples of the porous substrate include paper, such as filter paper, plain paper, premium paper, watercolor paper, Kent paper, and synthetic paper, that exhibits moderate voidage and hydrophilicity; however, the porous substrate is not limited to paper. The porous substrate may be synthetic resin porous film or cloth, a fiber product, or the like.
The channel region includes a working electrode disposing part (region A) and a reference electrode disposing part (region B). A region that connects the working electrode disposing part (region A) with the reference electrode disposing part (region B) may be provided, and a dispensing portion may be further provided.
Examples of the material of the channel wall include a hydrophobic resin; however, the material of the channel wall is not limited to a hydrophobic resin.
Examples of a method of forming the channel wall include a method of printing with an electrophotographic method using a hydrophobic resin as toner and melting and permeating the resin by heating. Examples of the resin include a thermoplastic resin.
The thermoplastic resin is not limited. Examples of thermoplastic resin include the following known resins. Examples of the thermoplastic resin include polyester resin, vinyl resin, acrylic resin, styrene acrylic resin, polyethylene, polypropylene, polyolefin, ethylene-vinyl acetate copolymer resin, and ethylene-acrylic acid copolymer resin. Among the above resins, polyester resin or styrene acrylic resin is preferable, and styrene acrylic resin is more preferable.
The method of forming a channel wall is not limited to the method of melting and permeating resin by heating and may be a method of permeating wax with a wax printer or a method of permeating resin by screen printing.

<Working Electrode Disposing Part>

The microchannel device has the working electrode disposing part (region A) in part of the channel region where a component having ion selectivity is present in a pore in the porous substrate. In the working electrode disposing part (region A), the component having ion selectivity is present in the pore at one plane X side of the porous substrate, and an ion selective membrane is formed. An other plane Y side of the porous substrate is a channel in which an empty pore is present.

A working electrode is provided on a surface at the plane X side of the ion selective membrane formed in the working electrode disposing part (region A).

The working electrode is preferably entirely in contact with the ion selective membrane at a surface opposed to the plane X side of the porous substrate in the region A. With such a configuration, direct contact of liquid having permeated into the channel with the working electrode without passing through the ion selective membrane can be suppressed, so only ions selected by the ion selective membrane can contact with the working electrode, and contact of other ions with the working electrode can be suppressed.

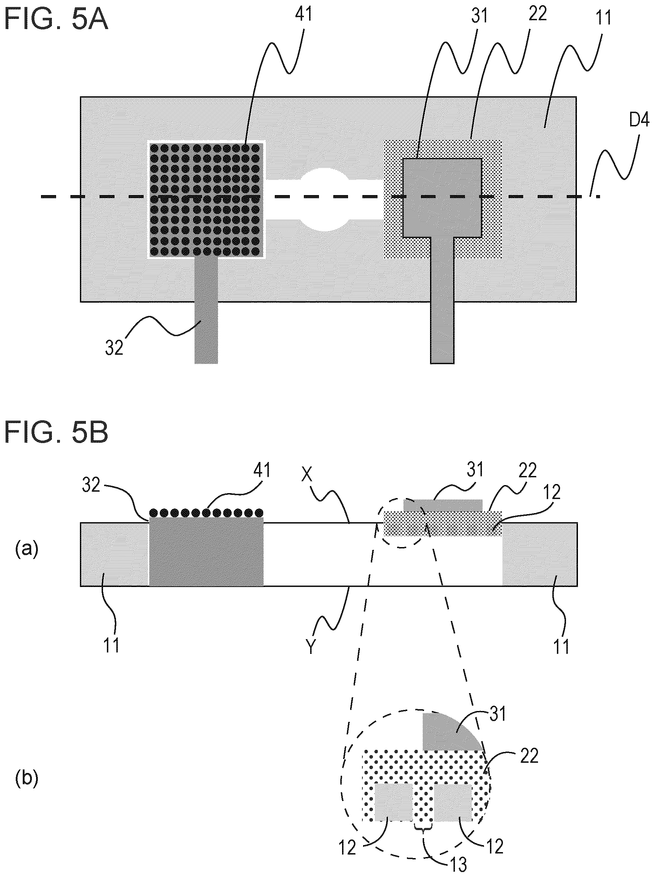

Preferably, the working electrode disposing part (region A) has a part (13 in [Fig. 5B] Fig. 5B ) that becomes a channel using a porous body and a part (12 in [Fig. 5B] Fig. 5B ) that is buried with a porous body so as not to function as a channel, at a surface of the plane X, and a part (ion selective membrane 22 in[Fig. 5B] Fig. 5B ) having ion selectivity is present so as to span between the part (13 in [Fig. 5B] Fig. 5B ) that becomes a channel and the part (12 in [Fig. 5B] Fig. 5B ) that does not function as a channel. This is to cause only ions selected by the ion selective membrane 22 to contact with the working electrode 31 and cause other ions not to contact with the working electrode 31.

<Reference Electrode Disposing Part>

A reference electrode is provided in the region B other than the region A of the channel region, that is, the reference electrode disposing part other than the working electrode disposing part.

[Example 1]

<Substrate / Channel>

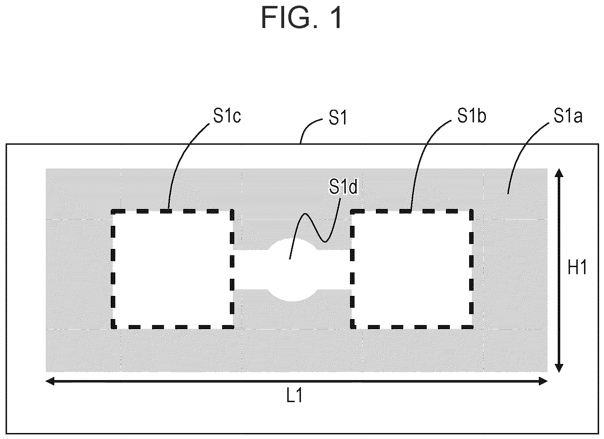

A substrate of the microchannel device M1 will be described with reference to Fig. 1. Fig. 1 schematically shows a top view of the microchannel device M1 before various electrodes are formed.

In the present embodiment, paper was used as a porous substrate S1. The material was cellulose, the thickness was 100 $\mu$m, and the voidage was 50%. Capillarity works due to micro air gaps between cellulose fibers, and the porous substrate S1 has high hydrophilicity and functions as a channel into which liquid smoothly permeates. A hydrophobic resin was caused to permeate into a region that is part of the porous substrate S1 to form a channel wall 11 with a height H1 = 10 mm and a width L1 = 22 mm. The channel wall 11 was formed by printing with an electrophotographic method using a hydrophobic resin as toner with the method described in Japanese Patent Laid-Open No. 2021-037612 and melting and permeating the resin by heating.

A region inside the channel wall 11 includes a region into which hydrophobic resin that becomes a channel wall is not caused to permeate, and this region becomes a channel using the porous body of the porous substrate S1. The channel is made up of a channel having a width of 2 mm and running from a working electrode disposing part S1b, a reference electrode disposing part S1c, and a dispensing portion S1d, or from the dispensing portion S1d to the working electrode disposing part S1b and a channel having a width of 2 mm and running from the dispensing portion S1d to the reference electrode disposing part S1c. A working electrode is disposed in the working electrode disposing part S1b, and a reference electrode is disposed in the reference electrode disposing part S1c. The dispensing portion S1d is located between the working electrode disposing part S1b and the reference electrode disposing part S1c. The working electrode disposing part S1b and the reference electrode disposing part S1c each have a square shape with 6 mm on each side, and the dispensing portion S1d has a circular shape with a diameter of 3 mm. The sizes, shapes, and the like of channels in Example 1 are as described above; however, the sizes, shapes, and the like of channels are not limited thereto.

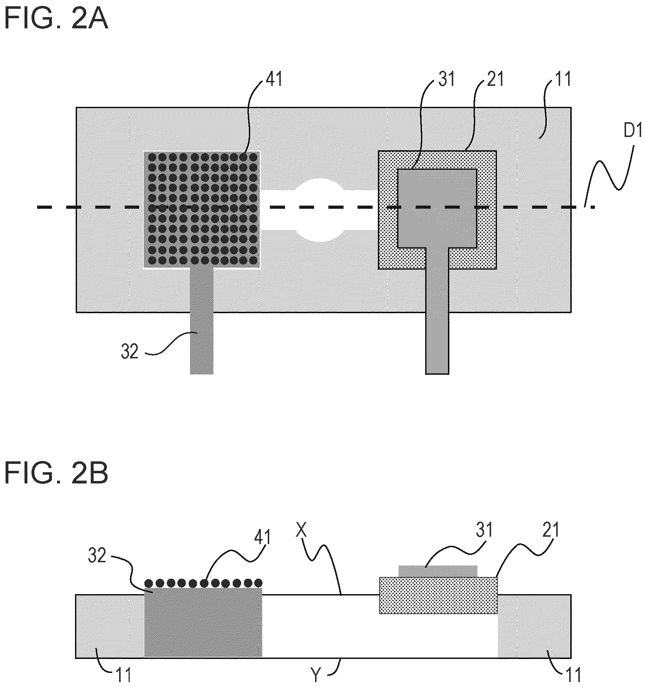

Hereinafter, formation of various components of the microchannel device M1 will be described with reference to Figs. 2A and 2B. Fig. 2A is a top view of the microchannel device M1 after various electrodes and the like are formed. Fig. 2B shows a cross section taken along the dashed line D1.

<Ion Selective Membrane>

As for an ion selective membrane 21, a membrane for selectively reacting with Na ions and measuring Na ion concentration was obtained by mixing materials described in the following Table 1, agitating the mixture until the mixture completely dissolves, and applying the dissolved mixture to the working electrode disposing part S1b. Material types and ratios are not limited thereto. A configuration just needs to have ion selectivity, so an ionophore according to an ion type to be measured or materials and a ratio suitable for the configuration may be selected as needed.

[Table 1]

| Ionophore | Bis[(12-crown-4)methyl]2-dodecyl-2-methylmalonate | 0.3wt% |
|---|---|---|
| Anion excluder | NaTFPB(Tetrakis[3,5-bis(trifluoromethyl)phenyl]borate, sodium salt) | 0.05wt% |
| Plasticizer | Dioctyl sebacate | 6.4wt% |
| Support | Polyvinyl chloride | 3.25wt% |
| Solvent | Cyclohexanone | 90wt% |

The ion selective membrane 21 was uniformly applied to the working electrode disposing part S1b with a reagent spotter Biospot made by MICROJET Co., Ltd. A discharge pipe diameter was $\Phi 200$ $\mu$m, a liquid droplet size was 4 nL/droplet, and discharge frequency was 4 Hz, and the film thickness was adjusted by applying five layers at a pitch of 300 $\mu$m. A stage for fixing the porous substrate S1 at the time of applying the ion selective membrane 21 was a heating stage and was set to a temperature or 90°C. When the solute concentration of a solution is set to 10mass%, the solution viscosity is relatively low, so stable discharge is easily performed. On the other hand, since the stage is heated, a solvent in the solution applied to the porous substrate S1 smoothly volatilizes, so the solution hardens before permeating into the porous substrate S1 in the thickness direction to bury the channel. Thus, even when the ion selective membrane 21 is applied to the X-side plane shown in Fig. 2B, the channel remains at the Y-side plane that is the back side from the X side, so the sample dispensed by the dispensing portion S1d can entirely permeate into the working electrode disposing part S1b. Heating of the stage is not indispensable. It is also possible to leave the channel at the Y-side plane by expanding the pitch between print dots, reducing print speed, or the like, other than reducing a liquid droplet size.

In this Example, the ion selective membrane 21 was applied with an inkjet method of discharging micro liquid droplets; however, the method is not limited thereto. For example, the ion selective membrane 21 may be applied with a dispenser or by screen printing. A dispenser or screen printing allows application even with a high viscosity as compared to the inkjet method, so it is possible to make it difficult for the ion selective membrane 21 to permeate a material for forming the ion selective membrane 21 into the porous substrate S1 in the thickness direction by increasing the viscosity through, for example, increasing the solute concentration.

<Working Electrode>

Ag was used as the material of the working electrode 31. Paste obtained by dispersing Ag in a hydrophobic solvent can form a good electrode without being rejected at the time of application onto the ion selective membrane 21 having similarly hydrophobicity. The material is not limited thereto. Any material is applicable as long as the material can be stably formed on the ion selective membrane 21 and functions as a working electrode.

In an application process for the working electrode 31, the ion selective membrane 21 was applied to the porous substrate S1, and then printed with a screen printing machine DP-320 made by NEWLONG MACHINE WORKS, LTD. A printing condition was #200, and drying was performed at a temperature of 80°C for 10 minutes.
A print shape was such that a square with 4 mm on each side in the ion selective membrane 21 (a square with 6 mm on each side) and a rectangle having a width of 1 mm and a length of 5 mm and extending from one side of the square with 4 mm on each side to the outer side of the channel wall 11 were connected. Then, the working electrode 31 can be connected to a measuring instrument that measures an electrode potential in a region outside the channel wall 11.

A solid-contact ion selective electrode was formed by directly printing the working electrode 31 on the ion selective membrane 21, so no air bubbles adhere to an electrode interface, and measurement can be stably performed.

<Reference Electrode>

Ag/AgCl was used as the material of the reference electrode 32. By using Ag/AgCl, an equilibrium reaction expressed by the following formula (1) takes place in the aqueous solution, so a stable potential can be obtained in a state where the concentration of Cl⁻ around the reference electrode 32 is stable.

[Formula 1]

$$\mathrm{Ag} + \mathrm{Cl}^- \rightleftarrows \mathrm{AgCl} + e^- \qquad \ldots \text{(Formula 1)}$$

The reference electrode 32 was applied to the reference electrode disposing part S1c with a screen printing machine DP-320 made by NEWLONG MACHINE WORKS, LTD. A printing condition was #200, and drying was performed at a temperature of 80°C for 10 minutes. The shape of the reference electrode 32 was such that a square with 6 mm on each side and a rectangle having a width of 1 mm and a length of 3 mm and extending from one side of the square with 6 mm on each side to the outer side of the channel wall 11 were connected. The reference electrode 32 can be connected to a measuring instrument that measures an electrode potential in a region outside the channel wall 11.

<Electrolyte Layer>

The role of an electrolyte layer 41 is to dissolve with the moisture of a sample when the sample permeates into the reference electrode disposing part S1c and saturates the concentration of Cl$^-$ to stabilize the potential of the reference electrode 32. KCl was used as the material therefor. KCl easily dissolves in water, the diffusion rate of K ions and the diffusion rate of Cl ions are substantially equal to each other and, therefore, a liquid junction potential is less likely to occur. For these reasons, KCl was selected. Of course, the material of the electrolyte layer 41 is not limited thereto. The material of the electrolyte layer 41 just needs to stabilize the potential of the reference electrode 32. For example, NaCl or the like may be used.

The electrolyte layer 41 was applied with a reagent spotter Biospot made by MICROJET Co., Ltd. A solution was prepared by dissolving KCl into pure water to have a concentration of 16wt%. The electrolyte layer 41 was entirely applied on the reference electrode disposing part S1c with a liquid droplet size of 6 nL/droplet, a discharge frequency of 10 Hz, at a pitch of 300 μm in both vertical and horizontal directions, and was applied in three layers to adjust the amount of KCl. The amount of KCl is set to an amount by which KCl saturates for the amount of sample that permeates into the reference electrode disposing part S1c, with the result that the potential of the reference electrode 32 can be stabilized. For this reason, as long as there is the amount of KCl by which saturated KCl can be prepared, the amount of application is not limited thereto.

<Permeation of Sample>

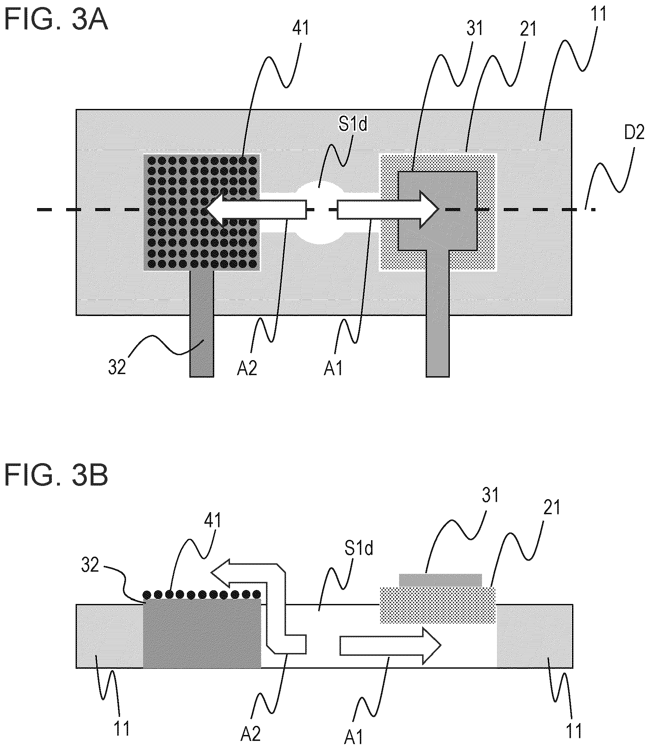

Permeation of a sample in the microchannel device M1 will be described with reference to Figs. 3A and 3B. Fig. 3A is a top view of the microchannel device M1 in which various electrodes are formed. Fig. 3B shows a cross section taken along the dashed line D2.

When the microchannel device M1 is used, a sample is dispensed to the dispensing portion S1d. Then, the dispensed sample flows along a channel due to capillarity and permeates in the direction of an arrow A1 and the direction of an arrow A2. At this time, as shown in Fig. 3B, the ion selective membrane 21 does not bury the porous substrate S1 in the thickness direction, so the sample can permeate into the working electrode disposing part S1b. The reference electrode 32 buries paper in the thickness direction, the sample having permeated to the reference electrode 32 continues to permeate into the reference electrode disposing part S1c while dissolving the electrolyte layer 41 applied to the surface of the reference electrode 32, and is held around the reference electrode 32 in a state where the concentration of Cl is saturated.

Thus, as described above, the reference electrode 32 stably exhibits a potential in a KCl saturated state regardless of a sample, while the working electrode 31 indicates a potential resulting from Nernst response of the ion selective membrane 21 according to the Na ion concentration of the sample. Thus, a potential difference between the reference electrode 32 and the working electrode 31 of the microchannel device M1 is measured, and the Na ion concentration of the sample can be detected with the ion electrode method by using the following formula (2).

$$E - E0 = 2.303 \times (RT/nF) \times \log(f \times c) \qquad \ldots \text{(2)}$$

E: electrode potential of working electrode
E0: electrode potential of reference electrode
R: gas constant (8.314 JK$^{-1}$mol$^{-1}$)
T: measurement temperature in absolute temperature
n: valence of measurement target ions
F: Faraday constant (9.649 × 10$^{-4}$ Gmol$^{-1}$)
f: activity coefficient

c: molar concentration of ions

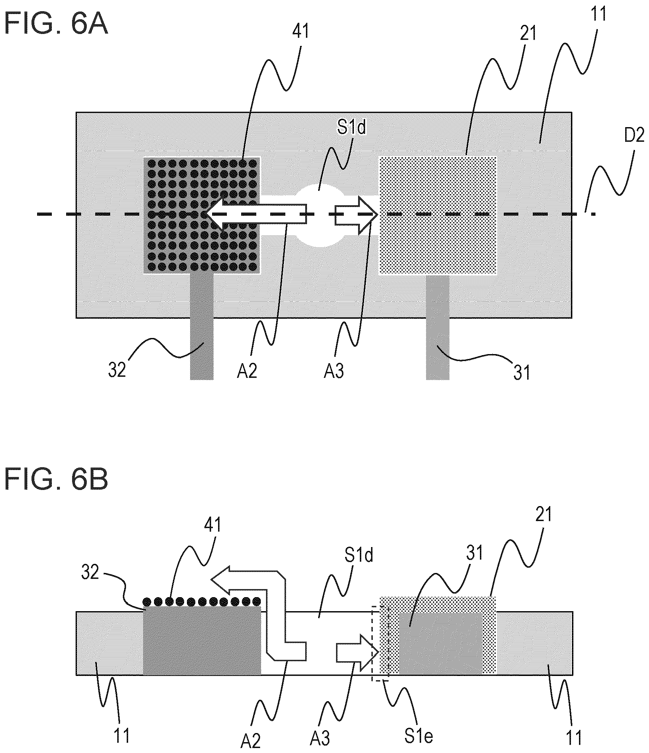

As described above, when the ion selective membrane 21 is applied so as not to completely bury the porous substrate S1 in the thickness direction and then the working electrode 31 is applied thereon, a contact area between the ion selective membrane 21 and the sample can be increased without increasing the area or perimeter of the ion selective membrane 21, so measurement is stable. For comparison, Figs. 6A and 6B show an existing configuration example in which the working electrode 31 is formed and then covered with the ion selective membrane 21. In the configuration of Figs. 6A and 6B, the ion selective membrane 21 entirely buries the porous substrate S1 in the thickness direction, so a sample dispensed to S1d only permeates to the part indicated by an arrow A3, and the sample and the ion selective membrane 21 contact with each other only at the interface of S1e, so a contact area is small. For this reason, with the configuration of this Example, it is possible to increase the contact area between the ion selective membrane 21 and the sample as compared to the existing configuration.

Contact between the ion selective membrane 21 and the working electrode 31 is solid contact not using an internal liquid therebetween, so air bubbles do not adhere to the surface of a base electrode, and measurement can be stably performed. Furthermore, since the ion selective membrane 21 and the working electrode 31 are configured on the single porous substrate, a thin film configuration is possible as compared to a case where an ion selective membrane is formed on a first porous substrate and a working electrode is formed on a second porous substrate disposed on the ion selective membrane.

[Example 2]

As for a microchannel device M2 according to this Example, only a difference from the microchannel device M1 of Example 1 will be described, and like reference signs denote the same members, and the description of similar parts is omitted.

<Channel>

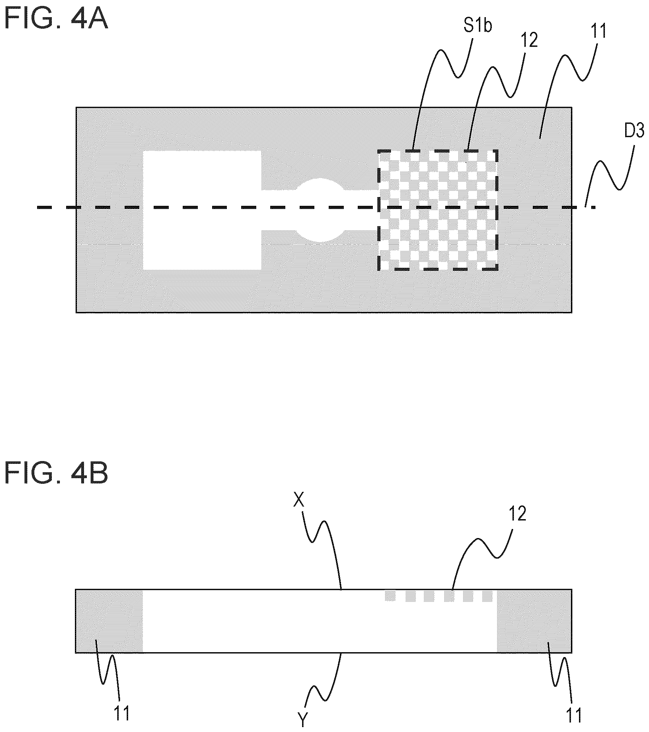

A channel of the microchannel device M2 will be described with reference to Figs. 4A and 4B. Fig. 4A schematically shows a top view of the microchannel device M2 before various electrodes are formed. Fig. 4B shows a cross section taken along the dashed line D3.

In the microchannel device M2, not only the channel wall 11 similar to that of the microchannel device M1 but also channel walls 12 are also formed in the working electrode disposing part S1b. The channel walls 12 have a checkerboard pattern with squares of 500 μm, hydrophobic resin is printed by electrophotography similar to Example 1, and can be easily printed by changing an image pattern. By reducing the print concentration of hydrophobic resin as compared to the channel wall 11, it is possible to perform adjustment such that the porous substrate S1 is not buried in the thickness direction. The phrase "checkerboard pattern with a square of 500 μm" means a state where, as shown in Fig. 4A, one side where a channel wall is formed at a location of a rectangle with 500 μm and one side where no channel wall is formed at a location of a rectangle with 500 μm are alternately arranged.

In this Example, the print concentration of the channel walls 12 was set to 20% of the print concentration of the channel wall 11. Thus, the X-side plane of the working electrode disposing part S1b is partially covered with resin, and a channel through which the sample permeates remains on the Y-side plane on the back side. Then, at a location where the channel walls 12 on the X-side plane are formed, the pore of the porous substrate S1 is buried with hydrophobic resin, so capillarity does not work, and liquid does not permeate. On the other hand, the pore of the porous substrate S1 remains at a location where there is no hydrophobic resin on the X-side plane (a location where the channel walls 12 are not formed), so such a location functions as a channel through which liquid permeates. By varying a ratio (hereinafter, also referred to as area ratio) of the area of the channel walls 12 (the total area of the channel walls 12) to the area of the working electrode disposing part S1b, it is possible to control the amount of liquid that permeates from the X-side plane into the porous substrate S1. In this Example, as shown in Fig. 4A, a checkerboard pattern is adopted, and the area ratio of the channel wall is 50%; however, the size and shape of each channel wall 12 and the area ratio are not limited thereto and are selectable as needed according to various configurations. The size and shape of each channel wall may vary according to the position of the channel wall (for example, a distance from the dispensing portion S1d).

Hereinafter, formation of the ion selective membrane 22 will be described with reference to Figs. 5A and 5B. Fig. 5A is a top view of the microchannel device M2 after various electrodes and the like are formed. Fig. 5B(a) shows a cross section taken along the dashed line D4. Fig. 5B(b) shows a partially enlarged view of Fig. 5B(a).

<Ion Selective Membrane>

The ion selective membrane 22 is made of a material similar to that of the ion selective membrane 21 and differs from the ion selective membrane 21 only in a substrate to which a material forming an ion selective membrane is applied. The ion selective membrane 22 was applied onto the channel walls 12 with a method similar to that of Example 1. At this time, since the ion selective membrane 22 permeates into the porous substrate S1 only from a part where there is no resin in the channel walls 12, the ion selective membrane 22 can be formed in a state where the porous substrate S1 reduces the amount of ion selective membrane to permeate and leaves more of channels in paper in the thickness direction as compared to Example 1. Thus, after a sample is dispensed into the dispensing portion S1d, the rate at which the sample permeates into the working electrode disposing part S1b increases, so it is possible to perform further quick measurement.

Other than the above, by forming the working electrode 31, the reference electrode 32, and the electrolyte layer 41 similarly to Example 1, the microchannel device M2 shown in Figs. 5A and 5B is obtained. Then, by measuring a potential difference between the reference electrode 32 and the working electrode 31 after a sample is dispensed to the dispensing portion S1d, it is possible to measure the Na ion concentration of the sample.

As described above, by forming in advance a channel wall in part of a channel in a region in which an ion selective membrane is formed, it is possible to control permeation of the ion selective membrane to a porous substrate in the thickness direction after being applied in the region, and it is possible to quickly perform measurement by further widely leaving the channel.

If the area ratio of the channel walls 12 is excessively increased, a contact area between the ion selective membrane 22 and a sample reduces, so the area ratio needs to be appropriately selected according to the size of the working electrode disposing part S1b, the thickness of the porous substrate, other desired measurement accuracy and rate, and the like. The area ratio of the channel walls 12 is not limited to the condition of this Example.

The present invention is not limited to the above-described embodiments. Various changes or modifications are applicable without departing from the spirit and scope of the present invention. Therefore, the following claims are attached to show the scope of the present invention.

This application claims the benefit of Japanese Patent Application No. 2022-074918 filed April 28, 2022, which is hereby incorporated by reference herein in its entirety.

Reference Signs List

| | |
|---|---|
| M1 | microchannel device |
| S1 | porous substrate |
| S1b | working electrode disposing part (region A) |
| S1c | reference electrode disposing part (region B) |
| S1d | dispensing portion |
| 11 | channel wall |
| 12 | channel wall |
| 21, 22 | ion selective membrane |
| 31 | working electrode |
| 32 | reference electrode |
| 41 | electrolyte layer |

**Claims**

1. A microchannel device using a porous substrate, wherein the microchannel device has a channel region using a porous body,

   the channel region partially has a region A where a component having ion selectivity is present in a pore in the porous substrate, and, in the region A, the component having ion selectivity is present in the pore at one plane X side of the porous substrate and an other plane Y side of the porous substrate is a channel in which an empty pore is present,
   a working electrode is provided on a surface at the plane X side of the region A so as to at least partially overlap a component having ion selectivity, and
   a reference electrode is provided in a region B other than the region A of the channel region, and the region A and the region B are formed in the same porous substrate.

2. The microchannel device according to Claim 1, wherein the working electrode is entirely in contact with the ion selective membrane at a surface opposed to the plane X side of the porous substrate in the region A.

3. The microchannel device according to Claim 1 or 2, wherein

   the region A has a part that becomes a channel using a porous body and a part that is buried with a porous body so as not to function as a channel, at a surface of the plane X, and
   the component having ion selectivity is present so as to span between the part that becomes a channel and the part that does not function as a channel.

4. The microchannel device according to Claim 3, wherein the part buried with a porous body so as not to function as a channel is a part of which a porous body is buried with hydrophobic resin so that the part does not function as a channel.

5. The microchannel device according to Claim 1 or 2, wherein the region A and the region B are connected via a channel.

# FIG. 1

FIG. 2A

FIG. 2B

FIG. 3A

FIG. 3B

# FIG. 4A

# FIG. 4B

FIG. 5A

FIG. 5B

FIG. 6A

FIG. 6B

## INTERNATIONAL SEARCH REPORT

| International application No. |
| --- |
| **PCT/JP2023/014603** |

| | |
| --- | --- |
| **A.** | **CLASSIFICATION OF SUBJECT MATTER** |

*G01N 27/333*(2006.01)i; *G01N 27/416*(2006.01)i; *G01N 37/00*(2006.01)i
FI: G01N27/333 331E; G01N27/416 346; G01N37/00 101

According to International Patent Classification (IPC) or to both national classification and IPC

| | |
| --- | --- |
| **B.** | **FIELDS SEARCHED** |

Minimum documentation searched (classification system followed by classification symbols)

G01N27/26-27/404; G01N27/414-27/416; G01N37/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JST7580 (JDreamIII)

| | |
| --- | --- |
| **C.** | **DOCUMENTS CONSIDERED TO BE RELEVANT** |

| Category* | Citation of document, with indication, where appropriate, of the relevant passages | Relevant to claim No. |
| --- | --- | --- |
| A | US 2016/0033438 A1 (PRESIDENT AND FELLOWS OF HARVARD COLLEGE) 04 February 2016 (2016-02-04)<br>entire text, all drawings | 1-5 |
| A | JP 2001-500259 A (USF FILTRATION AND SEPARATIONS GROUP INC) 09 January 2001 (2001-01-09)<br>entire text, all drawings | 1-5 |
| A | JP 9-229894 A (BAYER AG) 05 September 1997 (1997-09-05)<br>entire text, all drawings | 1-5 |
| A | ADKINS, Jaclyn et al. Electrochemical paper-based microfluidic devices. Electrophoresis. 27 March 2015, vol. 36, pp. 1811-1824, https://doi.org/10.1002/elps.201500084<br>entire text, all drawings | 1-5 |
| P, A | WO 2023/054095 A1 (CANON KK) 06 April 2023 (2023-04-06)<br>entire text, all drawings | 1-5 |

☐ Further documents are listed in the continuation of Box C.  ☑ See patent family annex.

| | |
| --- | --- |
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **06 June 2023** | **20 June 2023** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
Information on patent family members

International application No.

**PCT/JP2023/014603**

| Patent document cited in search report | | | Publication date (day/month/year) | Patent family member(s) | | | Publication date (day/month/year) |
|---|---|---|---|---|---|---|---|
| US | 2016/0033438 | A1 | 04 February 2016 | WO 2014/149611 A1 whole document | | | |
| JP | 2001-500259 | A | 09 January 2001 | US 6193865 B1 whole document WO 1998/011426 A1 EP 929804 A1 CA 2264288 A1 | | | |
| JP | 9-229894 | A | 05 September 1997 | US 5916156 A whole document EP 790498 A1 DE 19605583 A1 CA 2197385 A1 | | | |
| WO | 2023/054095 | A1 | 06 April 2023 | JP 2023-48923 A | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2016033438
- JP 2021037612 A
- JP 2022074918 A

**Non-patent literature cited in the description**

- **NIPAPAN RUECHA** ; **ORAWON CHAILAPAKUL** ; **KOJI SUZUKI** ; **DANIEL CHITTERIO**. Fully Inkjet-Printed Paper-Based Potentiometric Ion-Sensing Devices. *Analytical chemistry*, 29 August 2017, vol. 89, 10608-10616